(19) Europäisches Patentamt
European Patent Office
Office européen des brevets

(11) Veröffentlichungsnummer: **0 030 294**
**B1**

# (12) EUROPÄISCHE PATENTSCHRIFT

(45) Veröffentlichungstag der Patentschrift: **08.08.84**

(51) Int. Cl.³: **C 07 D 501/36,** C 07 D 501/04 // C07C131/00

(21) Anmeldenummer: **80107160.6**

(22) Anmeldetag: **18.11.80**

(54) **Verfahren zur Herstellung von Cephalosporinderivaten; Zwischenprodukte und deren Herstellung.**

(30) Priorität: **21.11.79 CH 10384/79**

(43) Veröffentlichungstag der Anmeldung: **17.06.81 Patentblatt 81/24**

(45) Bekanntmachung des Hinweises auf die Patenterteilung: **08.08.84 Patentblatt 84/32**

(84) Benannte Vertragsstaaten: **AT BE CH DE FR GB IT LI LU NL SE**

(56) Entgegenhaltungen:
**EP - A - 0 002 605**
**EP - A - 0 005 830**
**DE - A - 2 900 961**

**Die Akte enthält technische Angaben, die nach dem Eingang der Anmeldung eingereicht wurden und die nicht in dieser Patentschrift enthalten sind.**

(73) Patentinhaber: **F. HOFFMANN-LA ROCHE & CO. Aktiengesellschaft, CH-4002 Basel (CH)**

(72) Erfinder: **Montavon, Marc, Dr., Realpstrasse 72, CH-4054 Basel (CH)**
Erfinder: **Reiner, Roland, Dr., Rheinfelderstrasse 8, CH-4058 Basel (CH)**

(74) Vertreter: **Lederer, Franz, Dr. et al, Patentanwälte Dr. Franz Lederer Reiner F. Meyer Lucile-Grahn-Strasse 22, D-8000 München 80 (DE)**

**EP 0 030 294 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft ein Verfahren zur Herstellung von Acylderivaten, und zwar von Cephalosporinderivaten der allgemeinen Formel I

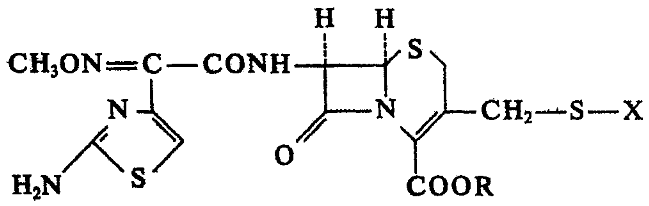

(I)

in der X eine der Gruppen

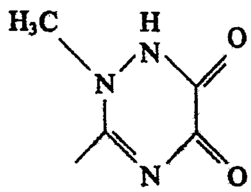

(a)

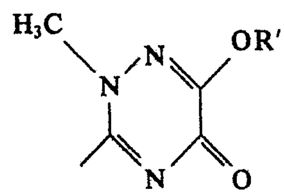

(b)

bedeutet und R Wasserstoff oder eine leicht hydrolysierbare Estergruppe und R′ Wasserstoff oder eine leicht hydrolysierbare Äthergruppe darstellt,
sowie von Salzen dieser Verbindungen und Hydraten dieser Verbindungen bzw. deren Salze.

Falls R′ in der Gruppe (b) Wasserstoff darstellt, liegt diese Gruppe in tautomerem Gleichgewicht mit der Gruppe (a) vor.

Als leicht hydrolysierbare Estergruppen R in den Verbindungen der Formel I sind R-Gruppen an der Carboxylfunktion zu verstehen, welche in Form einer leicht hydrolysierbaren Estergruppe vorliegen. Beispiele solcher Estergruppen, die herkömmlicher Art sein können, sind niederes Alkanoyloxyalkyl, z. B. Acetoxymethyl, Pivaloyloxymethyl, 1-Acetoxyäthyl und 1-Pivaloyloxyäthyl; niederes Alkoxycarbonyloxyalkyl, z. B. Methoxycarbonyloxymethyl, 1-Äthoxycarbonyloxyäthyl und 1-Isopropoxycarbonyloxyäthyl; Lactonylreste, z. B. Phthalidyl und Thiophthalidyl; niederes Alkoxymethyl, z. B. Methoxymethyl; und niederes Alkanoylaminomethyl, z. B. Acetamidomethyl.

Als leicht hydrolysierbare Äthergruppen R′ in den Verbindungen der Formel I sind R′-Gruppen an der enolischen Funktion der 2,5-Dihydro-6-hydroxy-2-methyl-5-oxo-as-triazin-3-ylgruppe (b) zu verstehen, welche in Form einer leicht hydrolysierbaren Äthergruppe vorliegen. Als Äthergruppen kommen die gleichen Gruppen in Betracht, wie sie oben bereits die leicht hydrolysierbaren Estergruppen erwähnt wurden. Vertreter solcher Äther sind also beispielsweise die niederen Alkanoyloxyalkyläther, z. B. der Acetoxymethyl-, Pivaloyloxymethyl-, 1-Acetoxyäthyl- und 1-Pivaloyloxyäthyläther; die niederen Alkoxycarbonyloxyalkyläther, z. B. der Methoxycarbonyloxymethyl-, 1-Äthoxycarbonyloxyäthyl- und 1-Isopropoxycarbonyloxyäthyläther; die Lactonyläther, z. B. der Phthalidyl- und Thiophthalidyläther; die niederen Alkoxymethyläther, z. B. der Methoxymethyläther; und die niederen Alkanoylaminomethyläther, z. B. der Acetamidomethyläther.

Beispiele von Salzen der Verbindungen der Formel I sind Alkalimetallsalze, wie das Natrium- und Kaliumsalz; das Ammoniumsalz; Erdalkalimetallsalze, wie das Calciumsalz; Salze mit organischen Basen, wie Salze mit Aminen, z. B. Salze mit N-Äthyl-piperidin, Procain, Dibenzylamin, N,N′-Dibenzyläthyläthylendiamin, Alkylaminen oder Dialkylaminen, sowie Salze mit Aminosäuren, wie z. B. Salze mit Arginin oder Lysin. Die Salze können Monosalze oder auch Disalze sein. Die zweite Salzbildung kann in Verbindungen mit dem Hydroxyrest der 2,5-Dihydro-6-hydroxy-2-methyl-5-oxo-as-triazin-3-ylgruppe auftreten.

Die Verbindungen der Formel I bilden ebenfalls Additionssalze mit organischen oder anorganischen Säuren. Beispiele solcher Salze sind Hydrohalogenide, beispielsweise Hydrochloride, Hydrobromide, Hydrojodide, sowie andere Mineralsäuresalze, wie Sulfate, Nitrate, Phosphate, Alkyl- und Mono-arylsulfonate, wie Äthansulfonate, Toluolsulfonate, Benzolsulfonate, und auch andere organische Säuresalze, wie Acetate, Tartrate, Maleate, Citrate, Benzoate, Salicylate und Ascorbate.

Die Verbindungen der Formel I (einschließlich deren Salze, leicht hydrolysierbare Ester und Äther) können hydratisiert sein. Die Hydratisierung kann im Zuge des Herstellungsverfahrens erfolgen oder allmählich als Folge hygroskopischer Eigenschaften eines zunächst wasserfreien Produktes auftreten.

Die erfindungsgemäßen Produkte können in der syn-isomeren Form (Z-Form)

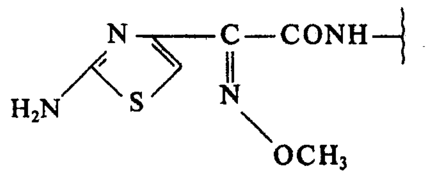

oder in der anti-isomeren Form (E-Form)

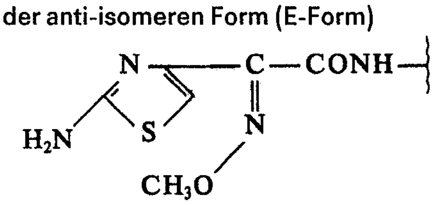

bzw. als Gemische dieser beiden Formen vorliegen. Bevorzugt ist die syn-isomere Form bzw. Gemische, in denen die syn-isomere Form überwiegt.

Bevorzugt sind (6R,7R)-7-[2-(Amino-4-thiazolyl)-2-(Z-methoxyimino)-acetamido]-3-/[(2,5-dihydro-6-hydroxy-2-methyl-5-oxo-as-triazin-3-yl)thio]-methyl/-8-oxo-5-thia-1-azabicyclo[4.2.0]oct-2-en-2-carbonsäure sowie ihre Salze und die entsprechenden Hydrate.

Die erfindungsgemäß herstellbaren Cephalosporine werden in der früheren, nicht vorveröffentlichten EP-A-5830 beschrieben; die vorliegende Erfindung betrifft ein zusätzliches Verfahren zu deren Herstellung. Ein ähnliches Verfahren wird in der DE-A-2 900 961 beschrieben, wobei jedoch die Endprodukte unterschiedliche Substitution in 3-Stellung aufweisen. Das erfindungsgemäße Verfahren basiert somit auf den gleichen erfinderischen Gedanken wie die Stofferfindung gemäß EP-A-5830.

Was die Endprodukte betrifft, so sind in der DE-A-2 715 385, der DE-A-2 707 565 und der DE-A-2 900 961 Cephalosporinderivate offenbart, welche, ähnlich wie die erfindungsgemäß herstellbaren Cephalosporine, eine 7-[2-(2-Amino-4-thiazolyl)-2-(Z-methoxyimino)-acetamido]-gruppe tragen. Die erfindungsgemäß herstellbaren Cephalosporine unterscheiden sich jedoch charakteristisch durch die Konfiguration des Substituenten in 3-Stellung, wodurch neue Verbindungen mit überraschenden Wirkungsvorteilen zur Verfügung gestellt werden.

Die Erfindung betrifft ein Verfahren zur Herstellung von Acylderivaten der allgemeinen Formel I

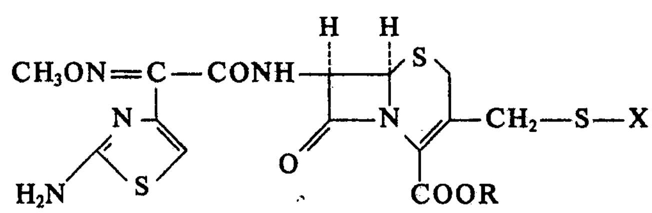

(I)

in der R Wasserstoff oder eine leicht hydrolysierbare Estergruppe darstellt,
sowie von Salzen dieser Verbindungen und Hydraten dieser Verbindungen bzw. Salze durch Umsetzung eines Halogenids der allgemeinen Formel II

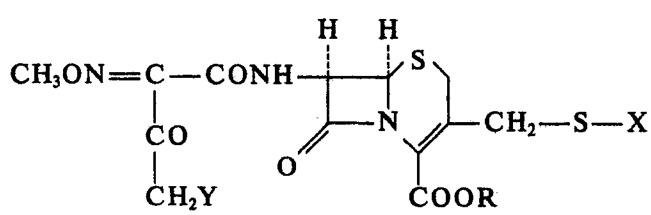

(II)

in der R die oben gegebene Bedeutung hat und Y ein Halogenatom darstellt,
oder eines Salzes dieser Verbindung mit Thioharnstoff und erwünschtenfalls Überführung einer erhaltenen Verbindung der Formel I in ein Salz oder Hydrat bzw. ein Hydrat dieses Salzes, dadurch gekennzeichnet, daß X eine der Gruppen

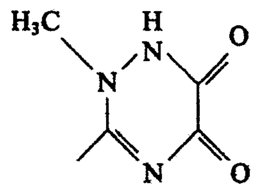

(a)

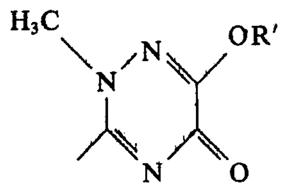

(b)

in der R' Wasserstoff oder eine leicht hydrolysierbare Äthergruppe darstellt, bedeutet.

Das erfindungsgemäße eingesetzte Halogenid der Formel II kann z. B. durch Umsetzung eines primären Amins der allgemeinen Formel

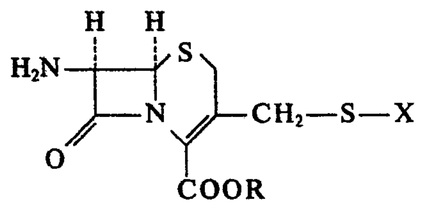

(III)

in der X und R die oben gegebene Bedeutung haben, mit einer halogenierten Carbonsäure der allgemeinen Formel IV

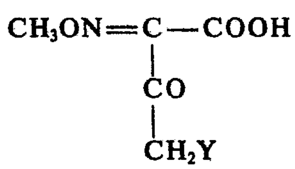

(IV)

in der Y ein Halogenatom darstellt, oder mit einem reaktionsfähigen Derivat dieser Verbindung hergestellt werden. Die halogenierte Carbonsäure der Formel IV wird entweder in freier Form eingesetzt in Gegenwart eines Kondensationsmittels, z. B. eines N,N'-disubstituierten Carbodiimides, wie N,N'-dicyclohexylcarbodiimid, oder einer Azolidverbindung, wie N,N'-Carbonyldiimidazol oder N,N'-Thionyldiimidazol oder auch in Form eines Säurehalogenids, wie des Säurechlorids oder -bromids, in Form eines Säureanhydrids, wie eines Säureanhydrids mit einem Kohlensäuremonoester, z. B. mit Monomethyl- oder Monoisopropylcarbonat, oder in Form eines aktivierten Esters, wie des p-Nitrophenylesters, 2,4-Dinitrophenylesters, N-Hydroxysuccinimidesters oder N-Hydroxyphthalimidesters. Die Reaktion erfolgt im allgemeinen in einem inerten organischen Lösungsmittel, z. B. in einem halogenierten Kohlenwasserstoff, wie Chloroform, Dichlormethan, Tetrachlorkohlenstoff, in einem Äther, z. B. Tetrahydrofuran, Dioxan, in Dimethylformamid, Dimethylacetamid, Wasser oder Mischungen davon. Die Reaktionstemperatur liegt vornehmlich im Bereich von −50 bis +40° C, vorzugsweise bei −10 bis +10° C.

Das erfindungsgemäß eingesetzte Halogenid der Formel II kann auch durch Halogenierung einer Verbindung der allgemeinen Formel V

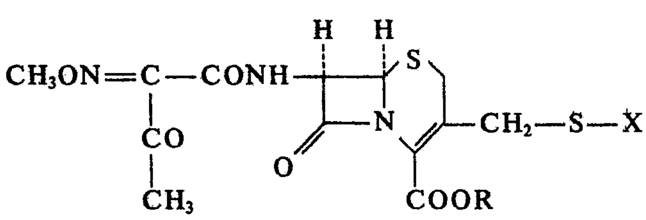

(V)

in der X und R die oben gegebene Bedeutung haben, hergestellt werden. Das hierbei verwendete Ausgangsprodukt der Formel V kann seinerseits durch ein Verfahren hergestellt werden, welches dadurch gekennzeichnet ist, daß man das oben definierte primäre Amin der Formel III mit 3-Oxo-2-methoximinobuttersäure oder mit einem reaktionsfähigen Derivat dieser Verbindung umsetzt. Diese Umsetzung verläuft im wesentlichen in der gleichen Weise wie die oben beschriebene Acylierung des primären Amins mit der halogenierten Carbonsäure der Formel IV bzw. mit einem reaktionsfähigen Derivat davon. Die Halogenierung des erhaltenen Acylierungsprodukts der Formel V führt zu den oben definierten Halogeniden der Formel II und erfolgt vorzugsweise durch Behandeln mit dem entsprechenden Halogen oder Thionylhalogenid, z. B. mit Chlor, Brom oder Sulfurylchlorid, bevorzugt in einem inerten Lösungsmittel, wie einem halogenierten

Kohlenwasserstoff, z. B. Dichlormethan, Dichloräthan, Chloroform, Dichloräthylen, Tetrachlorkohlenstoff, oder einer niederen Alkancarbonsäure, wie Essigsäure, einem aromatischen Lösungsmittel wie Benzol oder Toluol. Die Reaktionstemperatur liegt im allgemeinen zwischen 0° C und 60° C.

Die oben beschriebenen Zwischenprodukte der Formel II sowie deren Herstellung aus Vorprodukten der Formel III bzw. der Formel V sowie die Vorprodukte der Formel V und deren Herstellung ausgehend von Verbindungen der Formel III sind ebenfalls Gegenstand der vorliegenden Erfindung.

Die erfindungsgemäße Umsetzung des Halogenids der Formel II bzw. eines Salzes davon mit Thioharnstoff verläuft vorzugsweise in einem inerten Lösungsmittel, wie z. B. in einem niederen Alkanol, z. B. Äthanol, in einem niederen Keton, wie Aceton, in einem Äther, wie Tetrahydrofuran, Dioxan, in Dimethylformamid, Dimethylacetamid, in Wasser oder in Mischungen davon. Die Reaktionstemperatur liegt im allgemeinen im Bereich von 0°C bis 60°C, vorzugsweise bei Zimmertemperatur. Als Halogenid der Formel II kann das Chlorid, Bromid, Fluorid oder Jodid eingesetzt werden, bevorzugt verwendet man das Chlorid oder das Bromid. Es kann in freie Säure der Formel II eingesetzt werden, wahlweise aber auch ein Salz davon, wobei die gleichen Salze wie die oben erläuterten Salze der Verbindungen der Formel I in Betracht kommen.

Die Herstellung der Salze und Hydrate der Verbindungen der Formel I bzw. der Hydrate dieser Salze kann in an sich bekannter Weise erfolgen, z. B. durch Umsetzung der Carbonsäure der Formel I mit einer äquivalenten Menge der gewünschten Base, zweckmäßig in einem Lösungsmittel, wie Wasser oder in einem organischen Lösungsmittel, wie Äthanol, Methanol, Aceton und anderen mehr. Bei Verwendung eines zweiten Äquivalents an Base erfolgt Salzbildung auch an einer allenfalls vorhandenen tautomeren Enolform (2,5-Dihydro-6-hydroxy-2-methyl-5-oxo-as-triazin-3-ylgruppe X), wobei ein Disalz entsteht. Die Temperatur der Salzbildung ist nicht kritisch. Sie liegt im allgemeinen bei Raumtemperatur, kann aber auch, leicht darüber oder darunter, etwa im Bereiche von 0° C bis +50° C, sein.

Die Herstellung der Hydrate erfolgt zumeist automatisch im Zuge des Herstellungsverfahrens oder als Folge hydgroskopischer Eigenschaften eines zunächst wasserfreien Produktes. Zur gezielten Herstellung eines Hydrats kann ein ganz oder teilweise wasserfreies Produkt (Carbonsäure der Formel I bzw. Ester, Äther oder Salz davon) einer feuchten Atmosphäre, z. B. bei +10° C bis +40° C, ausgesetzt werden.

Ein allenfalls erhaltenes syn/anti-Gemisch einer Verbindung der Formel I kann in die entsprechenden syn- und anti-Formen in üblicher Weise aufgetrennt werden, beispielsweise durch Umkristallisation oder durch chromatographische Methoden unter Verwendung eines geeigneten Lösungsmittels bzw. Lösungsmittelgemisches.

Die Verbindungen der Formel I sowie die entsprechenden Salze bzw. die Hydrate dieser Produkte sind antibiotisch, insbesondere bakterizid wirksam. Sie besitzen ein breites Wirkungsspektrum gegen gram-positive und gram-negative Mikroorganismen, einschließlich β-Lactamase bildende Staphylokokken und verschiedene β-Lactamase-bildende gram-negative Bakterien, wie z. B. Pseudomonas aeruginosa, Haemophilus influenzae, Escherichia coli, Serratia marcescens, Proteus- und Klebsiella-Spezies.

Die Verbindungen der Formel I sowie die entsprechenden leicht hydrolysierbaren Ester, Äther und Salze bzw. die Hydrate dieser Produkte können zur Behandlung und Prophylaxe von Infektionskrankheiten verwendet werden. Für den Erwachsenen kommt eine Tagesdosis von 0,1 g bis 2 g in Betracht. Die parenterale Verabreichung der erfindungsgemäßen Verbindungen ist besonders bevorzugt.

Zum Nachweis der antimikrobiellen Wirksamkeit der erwähnten Produkte wurde die Aktivität in vitro (Mindesthemmkonzentration in µg/ml) ermittelt für (6R,7R)-7-[2-(2-Amino-4-thiazolyl)-2-(Z-methoxyimino)acetamido]-3-{[(2,5-dihydro-6-hydroxy-2-methyl-5-oxo-as-triazin-3-yl)thio]-methyl}-8-oxo-5-thia-1-azabicyclo[4.2.0]oct-2-en-2-carbonsäure (nachstehend mit A bezeichnet), einen repräsentativen Vertreter der erfindungsgemäß erhältlichen Verfahrensprodukte. Das Ergebnis ist in der nachstehenden Tabelle zusammengefaßt:

| Testorganismen | A |
|---|---|
| Haemophilus influenzae Stamm 1 | 0,08 |
| Stamm 2 | 0,005 |
| Stamm 3 | 0,005 |
| Stamm 4 | 0,005 |
| Stamm 5 | 0,0025 |
| Stamm 6 | 0,0025 |

| Testorganismen | A |
|---|---|
| Stamm 7 | 0,0025 |
| Klebsiella pneumoniae | 1,2 |
| Escherichia coli Stamm 1 | 0,02 |
| Stamm 2 | 0,6 |
| Proteus mirabilis Stamm 1 | < 0,01 |
| Stamm 2 | < 0,01 |
| Proteus vulgaris | < 0,01 |
| Proteus rettgeri | < 0,01 |
| Staphylococcus aureus Stamm ATCC 6538 | 2,5 |
| Penicillinresistenter Stamm | 2,5 |
| Pseudomonas aeruginosa Stamm 1 | 0,3 |
| Stamm 2 | 10 |
| Stamm 3 | 2,5 |
| Stamm 4 | 5 |
| Stamm 5 | 5 |
| Stamm 6 | 10 |
| Stamm 7 | 5 |
| Serratia marcescens | 0,08 |

Die antibakterielle Wirkung in vivo wurde wie folgt ermittelt:

Gruppen von 5 Mäusen werden mit einer wäßrigen Suspension von Escherichia coli intraperitoneal infiziert. Dreimal, d. h. 1 Stunde, $2^1/_2$ Stunden und 4 Stunden nach der Infektion, wird die Prüfsubstanz in physiologischer Kochsalzlösung subcutan appliziert. Die Zahl der überlebenden Tiere wird am 4. Tag bestimmt. Es werden verschiedene Dosierungen appliziert, und durch Interpolation wird diejenige Dosis bestimmt, bei der 50% der Versuchstiere überleben ($CD_{50}$, mg/kg).

| Prüfsubstanz | A |
|---|---|
| $CD_{50}$, mg/kg | 0,005 |

Toxizität

| Prüfsubstanz | A |
|---|---|
| $LD_{50}$, mg/kg i. v. | 250–500 |
| s. c. | > 4000 |
| p. o. | > 5000 |

Die erfindungsgemäß erhältlichen Produkte können als Heilmittel z. B. in Form pharmazeutischer Präparate Verwendung finden, welche sie oder ihre Salze in Mischung mit einem für die enterale oder parenterale Applikation geeigneten pharmazeutischen, organischen oder anorganischen inerten Trägermittel, wie z. B. Wasser, Gelatine, Gummi arabicum, Milchzucker, Stärke, Magnesiumstearat, Talk, pflanzliche Öle, Polyalkylenglykole, Vaseline usw., enthalten. Die pharmazeutischen Präparate können in fester Form, z. B. als Tabletten, Dragées, Suppositorien, Kapseln; oder in flüssiger Form, z. B. als Lösungen, Suspensionen oder Emulsionen, vorliegen. Gegebenenfalls sind sie sterilisiert und bzw. oder enthalten Hilfsstoffe, wie Konservierungs-, Stabilisierungs-, Netz- oder Emulgiermittel, Salze zur Veränderung des osmotischen Druckes, Anaesthetica oder Puffer. Sie können auch noch andere therapeutische wertvolle Stoffe enthalten. Die Verbindungen der Formel I und ihre Salze bzw. Hydrate kommen vorzugsweise für die parenterale Applikation in Betracht und werden zu diesem Zweck bevorzugt als Lyophilisate oder Trockenpulver zur Verdünnung mit üblichen Agenzien, wie Wasser oder isotonische Kochsalzlösung, zubereitet. Die leicht hydrolysierbaren Ester bzw. Äther der Verbindungen der Formel I und ihre Salze bzw. Hydrate kommen auch für die enterale Verabreichung in Betracht.

Beispiel 1

28,8 g (6R,7R)-7-{4-Brom-2-[(Z)-methoxyimino]acetoacetamido}-3-{[(2,5-dihydro-6-hydroxy-2-methyl-5-oxo-as-triazin-3-yl)thio]methyl}-8-oxo-5-thia-1-azabicycle [4.2.0]oct-2-en-2-carbonsäure werden in 400 ml absolutem Äthanol gelöst. Die Lösung wird mit 7,6 g Thioharnstoff versetzt. Nach 15—20 Minuten Rühren bei 25°C beginnt aus der orangen Lösung das Reaktionsprodukt als Hydrobromid zu kristallisieren. Nach 1$^1/_2$ Stunden Rühren wird letzteres abgenutscht, nacheinander mit 200 ml Alkohol und 200 ml tiefsiedendem Petroläther gewaschen, und über Nacht im Hochvakuum bei 35—40°C getrocknet. Man erhält 22 g praktisch farbloses Hydrobromid. Dieses wird in einem Gemisch von 110 ml Wasser und 110 ml Aceton zusammen mit 15,7 g Natriumacetat-trihydrat gelöst. Zu dieser Lösung werden 140 ml Aceton bis zur leichten Trübung hinzugefügt. Kurz darauf beginnt das Reaktionsprodukt zu kristallisieren. Das Gemisch wird 30 Minuten gerührt, danach werden während weiteren 30 Minuten 180 ml Aceton zugetropft, wobei die Kristallisation vervollständigt wird. Das Reaktionsprodukt wird abgenutscht, nacheinander mit 250 ml 85%igem wäßrigem Aceton, 250 ml Aceton und 250 ml tiefsiedendem Petroläther gewaschen und schließlich im Vakuum bei 25°C getrocknet. Man erhält 19,6 g (59,3% d. Th.) praktisch farbloses Dinatriumsalz der (6R,7R)-7-[2-(2-Amino-4-thiazolyl)-2-(Z-methoxyimino)acetamido]-3-{[(2,5-dihydro-6-hydroxy-2-methyl-5-oxo-as-triazin-3-yl)thio]-methyl}-8-oxo-5-thia-1-azabicyclo[4.2.0]oct-2-en-carbonsäure, welches aus Wasser-Aceton umkristallisiert wird. Man erhält 17,3 g (52,3% d. Th.) Reinsubstanz mit $[\alpha]_D^{25} = -150{,}8°$ (c=1 in Wasser). 1H-NMR-Spektrum in DMSO-$d_6$ ($\delta$-Werte in ppm, s=Singlett, d=Dublett, q=Quartett, b=breit; J=Kopplungskonstante in Hz; Protonenzahl in Klammern): 3,47 ($N-CH_3$) (s) (3), 3,49 (2-$CH_2$) (AB—q/Zentrierungswert; $J_{gem} = \sim 18$ Hz) (2),

$$3{,}85 \left[=N{\diagup}^{OCH_3}\ ,\ (Z)\right] (s)\ (3),$$

4,37 ($3-CH_2-S$) (AB—q/Zentrierungswert; $J_{gem} = 12{,}5$ Hz) (2), 5,04 (H-6) (d, $J_{H-6,H-7} = 4{,}5$ Hz) (1), 5,59 (H—7) (q, $J_{H-7,NH} = 8$ Hz, $J_{H-7,H-6} = 4{,}5$ Hz) (1), 6,75 (Thiazolyl-H) (s) (1), 7,29 ($-NH_2$) (b) (2), 9,52 ($-CO-NH-$) (d, $J_{NH,H-7} = 8$ Hz) (1).

Die als Ausgangsverbindung eingesetzte (6R,7R)-7-{4-Brom-2-[(Z)-methoxyimino]acetoacetamido}-3-{[[(2,5-dihydro-6-hydroxy-2-methyl-5-oxo-as-triazin-3-yl)thio]methyl}-8-oxo-5-thia-1-azabicyclo[4.2.0]oct-2-en-2-carbonsäure kann wie folgt hergestellt werden:

592,1 g Acetessigsäure-tert.-butylester werden in 560 ml Eisessig gelöst. Zu dieser Lösung wird bei 5—10°C während 2$^1/_2$ Stunden eine Lösung von 290,6 g Natriumnitrit in 655 ml Wasser getropft. Die entstandene gelbe Suspension wird 30 Minuten bei 20°C gerührt, mit 940 ml Wasser versetzt und weitere 2 Stunden gerührt. Das Gemisch wird mit 900 ml Wasser und 900 g Eis versetzt und im Ausrührgefäß dreimal mit je 1 l Äthylacetat extrahiert. Die vereinigten Äthylacetatextrakte werden dreimal mit je 1 l Wasser gewaschen, dann mit 5 l Wasser versetzt und mit Natriumhydrogencarbonat der pH-Wert auf 6,8 gestellt. Nach dem Abtrennen der wäßrigen Phase wird noch einmal mit Wasser gewaschen. Danach wird die Äthylacetatlösung über Natriumsulfat getrocknet und am Vakuum bei 40°C eingedampft. Man erhält (Z)-2-Hydroxyimino-3-oxo-buttersäure-tert.-butylester als gelbes Öl, das noch 9 Stunden am Hochvakuum bei 40°C getrocknet wird. Die Ausbeute beträgt 626,65 g (89,2% d. Th.).

626,65 g (Z)-2-Hydroxyimino-3-oxo-buttersäure-tert.-butylester werden in 2,86 l Aceton gelöst. Die Lösung wird auf 5°C abgekühlt und portionsweise mit 703,5 g Kaliumcarbonat versetzt. Zur gelben Suspension werden dann ohne Kühlung während 1 Stunde 322 ml Dimethylsulfat hinzugetropft, wobei die Temperatur des Gemisches nicht über 25°C steigen soll. Die hellbeige Suspension wird bei

20–25°C ca. 4 Stunden gerührt, bis dünnschicht-chromatographisch kein Ausgangsmaterial mehr nachgewiesen wird. Danach wird das Gemisch auf 7 l Wasser gegossen und dreimal mit je 1 l Äthylacetat extrahiert. Die vereinigten Äthylacetatextrakte werden dreimal mit je 1 l Wasser gewaschen, über Natriumsulfat getrocknet und am Vakuum bei 40°C eingedampft. Das verbleibende, gelbe Öl wird noch 6 Stunden am Hochvakuum bei 40°C getrocknet und anschließend destilliert. Man erhält 577 g (Z)-2-Methoxyimino-3-oxo-buttersäure-tert.-butylester (85,6% d. Th.) als gelbes Öl mit einem Siedepunkt von 57°C bei 0,02 mm Hg. $^1$H-NMR-Spektrum in $CDCl_3$ ($\delta$-Werte in ppm, s = Singlett, Protonenzahl in Klammern): 1,55 [$-C(CH_3)_3$] (s) (9), 2,36 ($CH_3-CO$) (s) (3),

4,01 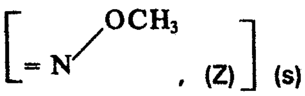 (Z) (s) (3).

86 g (Z)-2-Methoxyimino-3-oxo-buttersäure-tert.-butylester werden in 400 ml Trifluoressigsäure gelöst. Die Lösung wird 1 Stunde bei 25°C stehengelassen, danach am Vakuum bei 35°C eingedampft. Der ölige Rückstand wird aus Äther/Petroläther kristallisiert. Man erhält 50 g (80,6% d. Th.) gelbliche, wasserlösliche (Z)-2-Methoxyimino-3-oxo-buttersäure vom Smp. 80–85°C. $^1$–H–NMR-Spektrum in $CDCl_3$ ($\delta$-Werte in ppm, s = Singlett, Protonenzahl in Klammern): 2,43 ($CH_3-CO$) (3),

4,14 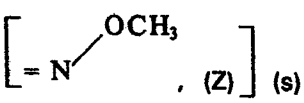 (Z) (s) (3),

10,47 (OH) (s) (1).

145 g (Z)-2-Methoxyimino-3-oxo-buttersäure werden in 1000 ml alkohol- und wasserfreiem Dichlormethan gelöst. Zu dieser Lösung werden 10 ml 30%ige Bromwasserstoffsäure in Eisessig gegeben. Dann wird während ca. 2 Stunden eine Lösung von 37,5 ml Brom in 112,5 ml Dichlormethan zugetropft, wobei die Temperatur des Reaktionsgemisches mittels leichter Kühlung auf 20–25°C gehalten wird. Nun wird zur Entfernung von HBr aus dem Reaktionsgemisch heftig Stickstoff durchgeblasen. Anschließend werden nacheinander 250 g Eis, 250 ml Wasser und 2 l Äther zugegeben. Die wäßrige Phase wird abgetrennt und verworfen. Die organische Phase wird mit 250 ml Wasser und 250 ml gesättigter Natriumchloridlösung gewaschen, mit Natriumsulfat getrocknet und im Vakuum eingedampft. Es verbleiben 170 g braunes Öl, das aus Tetrachlorkohlenstoff kristallisiert wird. Man erhält 100 g (44,6% d. Th.) praktisch farblose (Z)-4-Brom-2-methoxyimino-3-oxo-buttersäure $^1$H–NMR-Spektrum in $CDCl_3$($\delta$-Werte in ppm, S = Singlett, Protonenzahl in Klammern):

4,24 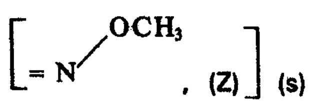 (Z) (s) (3),

4,41 ($-CH_2-$) (s) (2), 11,00 (OH) (s) (1).

37,1 g (7R)-7-Amino-3-desacetoxy-3-[(2,5-dihydro-6-hydroxy-2-methyl-5-oxo-as-triazin-3-yl)-thio]-cephalosporansäure werden in 800 ml Essigester suspendiert und mit 100 ml N,O-Bis-(trimethylsilyl)-acetamid versetzt. Das Gemisch wird unter Feuchtigkeitsausschluß 30 Minuten bei 25°C gerührt, wobei eine hellgelbe Lösung entsteht. Dieser auf –10°C gekühlten Lösung wird eine Lösung von (Z)-4-Brom-2-methoxyimino-3-oxo-buttersäurechlorid, welches aus 22,4 g (Z)-4-Brom-2-methoxyimino-3-oxo-buttersäure in 300 ml alkohol- und wasserfreiem Dichlormethan und 20,8 g Phosphorpentachlorid bei 8–10°C hergestellt worden war, während 30 Minuten bei minus 10 bis 0°C zugetropft. Das Reaktionsgemisch wird 30 Minuten bei 0–5°C und 1 Stunde bei 25°C gerührt. Es werden 1 l Essigester und 500 ml Wasser unter Rühren hinzugefügt. Die wäßrige Phase und die harzige Zwischenschicht werden verworfen. Die organische Phase wird sechsmal mit je 500 ml Wasser gewaschen, mit Natriumsulfat getrocknet und am Vakuum bei 40°C auf ein Volumen von 200 ml eingeengt. Dieses orange gefärbte Konzentrat wird unter Rühren zu 1,8 l Äther getropft, wobei das Reaktionsprodukt amorph ausfällt. Dieses wird abgenutscht, nacheinander mit 1 l Äther und 1 l tiefsiedendem Petroläther gewaschen und über Nacht im Vakuum bei 35°C getrocknet. Man erhält 37,2 g (64,4% d. Th.) (6R,7R)-7-{4-Brom-2-[(Z)-methoxyimino]acetoacetamido}-3-{[(2,5-dihydro-6-hydroxy-2-methyl-5-oxo-as-triazin-3-yl)thio]methyl}-8-oxo-5-thia-1-azabicyclo[4.2.0]oct-2-en-2-carbonsäure als beiges, amorphes Produkt mit $[\alpha]_D^{25} = -223,1°$ (c = 1 in Methanol. $^1$H–NMR-Spektrum in DMSO-$d_6$ ($\delta$-Werte in ppm, s = Singlett, d = Dublett, q = Quartett, b = breit; J = Kopplungskonstante in Hz; Protonenzahl in Klammern): 3,60 ($N-CH_3$) (s) (3), 3,63 ($2-CH_2$) (AB–q/Zentrierungswert; $J_{gem}$ = 18 Hz) (2),

4,07 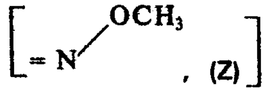 (Z) (s) (3),

4,25 (3—$CH_2$—S) (AB—q/Zentrierungswert; $J_{gem}$ = 13 Hz) (2), 4,62 (Br—$CH_2$—) (S) (2), 5,15 (H—6) (d,$J_{H-6,H-7}$ = 5 Hz) (1), 5,78 (H—7) (q,$J_{H-7,NH}$ = 8 Hz, $J_{H-7,H-6}$ = 5 Hz) (1), 9,47 (NH) (d,$J_{NH,H-7}$ = 8 Hz) (1), 11,12 (—COOH und —OH) (b) (2).

## Beispiel 2

Herstellung von Trockenampullen für die intramuskuläre Verabreichung

Es wird in üblicher Weise ein Lyophilisat von 1 g des Dinatriumsalzes der (6R,7R)-7-[2-2-amino-4-thiazolyl)-2-(Z-methoxyimino)acetamido]-3-{[(2,5-dihydro-6-hydroxy-2-methyl-5-oxo-as-triazin-3-yl)thio]methyl}-8-oxo-5-thia-1-azabicyclo[4.2.0]oct-2-en-2-carbonsäure hergestellt und in eine Ampulle abgefüllt. Vor der Verabreichung wird das Lyophilisat mit 2,5 ml einer 2%igen wäßrigen Lidocainhydrochlorid-Lösung versetzt.

**Patentansprüche für die Vertragsstaaten: RE, CH, DE, FR, GB, IT, LI, LU, NL, SE**

1. Verfahren zur Herstellung von Acylderivaten der allgemeinen Formel I

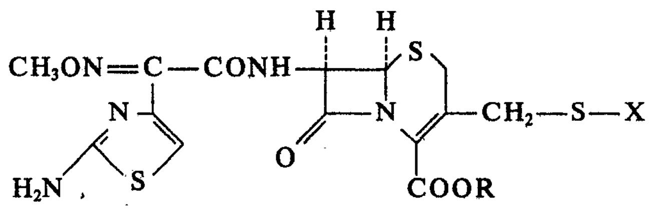

(I)

in der R Wasserstoff oder eine leicht hydrolysierbare Estergruppe darstellt,
sowie von Salzen dieser Verbindungen und Hydraten dieser Verbindungen bzw. deren Salze durch Umsetzung eines Halogenids der allgemeinen Formel II

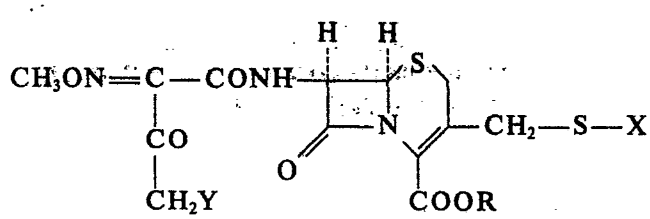

(II)

in der R die oben gegebene Bedeutung hat und Y ein Halogenatom darstellt,
oder eines Salzes dieser Verbindung mit Thioharnstoff und erwünschtenfalls Überführung einer erhaltenen Verbindung der Formel I in ein Salz oder Hydrat bzw. ein Hydrat dieses Salzes, dadurch gekennzeichnet, daß X eine der Gruppen

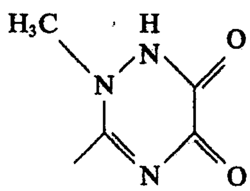

(a)

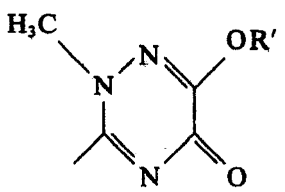

(b)

in der R' Wasserstoff oder eine leicht hydrolysierbare Äthergruppe darstellt,
bedeutet.

2. Verfahren nach Anspruch 1, worin R und R' Wasserstoff darstellt.
3. Verfahren nach Anspruch 1 oder 2, worin Y Brom darstellt.
4. Verfahren nach einem der Ansprüche 1—3, worin die Ausgangsverbindung der Formel II in der syn-Form (Z-Form) vorliegt.

5. Verfahren nach einem der Ansprüche 1—4, worin das Halogenid der Formel II durch Umsetzung eines primären Amins der allgemeinen Formel III

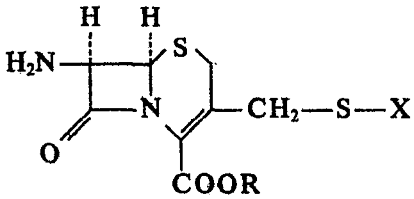

(III)

in der X und R die in Anspruch 1 gegebene Bedeutung haben, mit einer halogenierten Carbonsäure der allgemeinen Formel IV

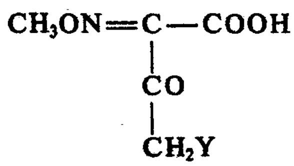

(IV)

in der Y ein Halogenatom darstellt, oder mit einem reaktionsfähigen Derivat dieser Verbindung hergestellt worden ist.

6. Verfahren nach Anspruch 5, worin R und R' Wasserstoff darstellt.

7. Verfahren nach Anspruch 5 oder 6, worin Y Brom darstellt.

8. Verfahren nach einem der Ansprüche 5—7, worin die halogenierte Carbonsäure der Formel IV bzw. das reaktionsfähige Derivat davon in der syn-Form (Z-Form) vorliegt.

9. Verfahren nach einem der Ansprüche 5—8, worin man ein Säurechlorid der halogenierten Carbonsäure der Formel IV verwendet.

10. Verfahren nach einem der Ansprüche 1—4, worin das Halogenid der Formel II durch Halogenierung einer Verbindung der allgemeinen Formel V

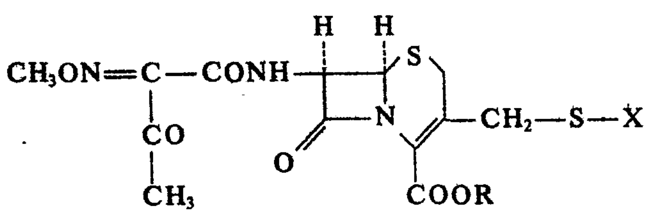

(V)

in der X und R die in Anspruch 1 gegebene Bedeutung haben, hergestellt worden ist.

11. Verfahren nach Anspruch 10, worin R und R' Wasserstoff darstellt.

12. Verfahren nach Anspruch 10 oder 11, worin die Verbindung der Formel V in der syn-Form (Z-Form) vorliegt.

13. Verbindungen der allgemeinen Formel

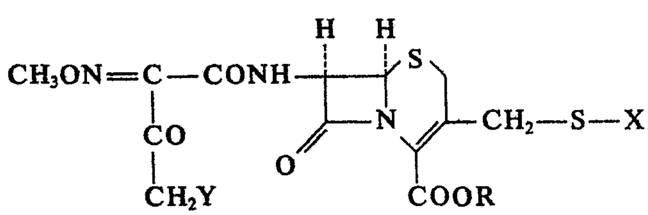

(II)

in der Y ein Halogenatom und X eine der Gruppen

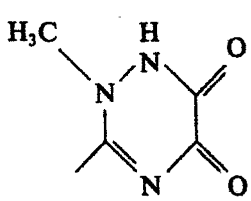

(a)

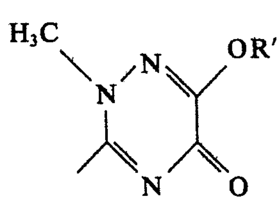

(b)

bedeutet und R Wasserstoff oder eine leicht hydrolysierbare Estergruppe und R' Wasserstoff oder eine leicht hydrolysierbare Äthergruppe darstellt,
sowie deren Salze.

14. Verbindungen gemäß Anspruch 13, dadurch gekennzeichnet, daß R und R' Wasserstoff darstellt.

15. Verbindungen gemäß Anspruch 13 oder 14, dadurch gekennzeichnet, daß Y Brom darstellt.

16. (6R,7R){4-Brom-2-[(Z)-methoxyimino]acetoacetamido}-3-{[(2,5-dihydro-6-hydroxy-2-methyl-5-oxo-as-triazin-3-yl)thio]methyl}-8-oxo-5-thia-1-azabicyclo[4.2.0]oct-2-en-2-carbonsäure und deren Salze.

**Patentansprüche für den Vertragsstaat: AT**

1. Verfahren zur Herstellung von Acylderivaten der allgemeinen Formel I

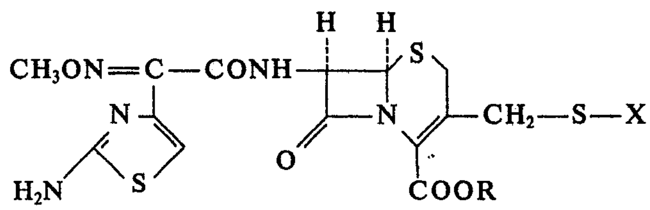

(I)

in der R Wasserstoff oder eine leicht hydrolysierbare Estergruppe darstellt,
sowie von Salzen dieser Verbindungen und Hydraten dieser Verbindungen bzw. deren Salze durch Umsetzung eines Halogenids der allgemeinen Formel II

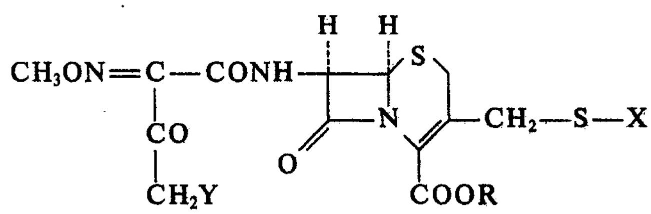

(II)

in der R die oben gegebene Bedeutung hat und Y ein Halogenatom darstellt,
oder eines Salzes dieser Verbindung mit Thioharnstoff und erwünschtenfalls Überführung einer erhaltenen Verbindung der Formel I in ein Salz oder Hydrat bzw. ein Hydrat dieses Salzes, dadurch gekennzeichnet, daß X eine der Gruppen

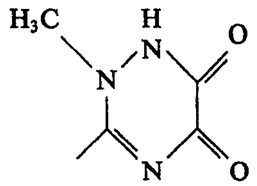

(a)

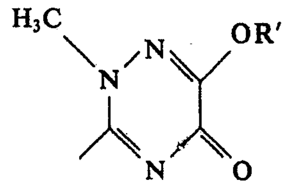

(b)

in der R' Wasserstoff oder eine leicht hydrolysierbare Äthergruppe darstellt,
bedeutet.

2. Verfahren nach Anspruch 1, worin R und R' Wasserstoff darstellt.

3. Verfahren nach Anspruch 1 oder 2, worin Y Brom darstellt.

4. Verfahren nach einem der Ansprüche 1—3, worin die Ausgangsverbindung der Formel II in der syn-Form (Z-Form) vorliegt.

5. Verfahren nach einem der Ansprüche 1—4, worin das Halogenid der Formel II durch Umsetzung eines primären Amins der allgemeinen Formel III

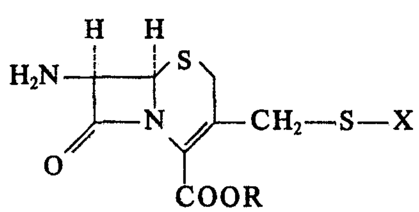

(III)

in der X und R die in Anspruch 1 gegebene Bedeutung haben, mit einer halogenierten Carbonsäure der allgemeinen Formel IV

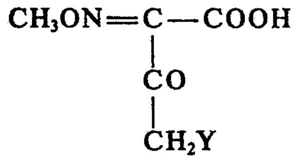

(IV)

in der Y ein Halogenatom darstellt, oder mit einem reaktionsfähigen Derivat dieser Verbindung hergestellt worden ist.

6. Verfahren nach Anspruch 5, worin R und R' Wasserstoff darstellt.

7. Verfahren nach Anspruch 5 oder 6, worin Y Brom darstellt.

8. Verfahren nach einem der Ansprüche 5—7, worin die halogenierte Carbonsäure der Formel IV bzw. das reaktionsfähige Derivat davon in der syn-Form (Z-Form) vorliegt.

9. Verfahren nach einem der Ansprüche 5—8, worin man ein Säurechlorid der halogenierten Carbonsäure der Formel IV verwendet.

10. Verfahren nach einem der Ansprüche 1—4, worin das Halogenid der Formel II durch Halogenierung einer Verbindung der allgemeinen Formel V

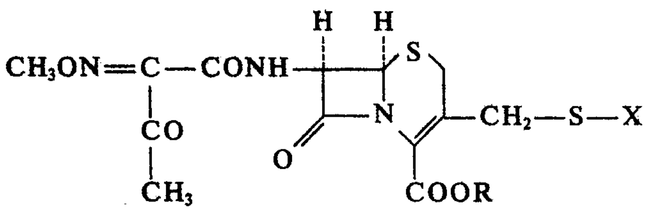

(V)

in der X und R die in Anspruch 1 gegebene Bedeutung haben, hergestellt worden ist.

11. Verfahren nach Anspruch 10, worin R und R' Wasserstoff darstellt.

12. Verfahren nach Anspruch 10 oder 11, worin die Verbindung der Formel V in der syn-Form (Z-Form) vorliegt.

**Claims for the Contracting States: BE, CH, DE, FR, GB, IT, LI, LU, NL, SE**

1. A process for the manufacture of acyl derivatives of general formula I

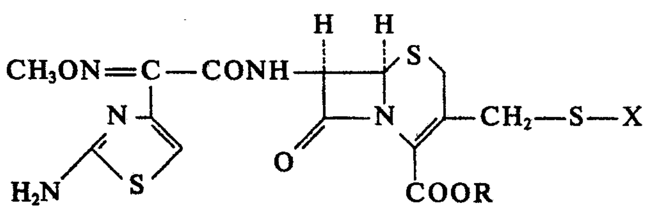

(I)

in which R represents hydrogen or a readily hydrolyzable ester group, as well as of salts of these compounds and hydrates of these compounds or of their salts by reacting a halide of general formula II

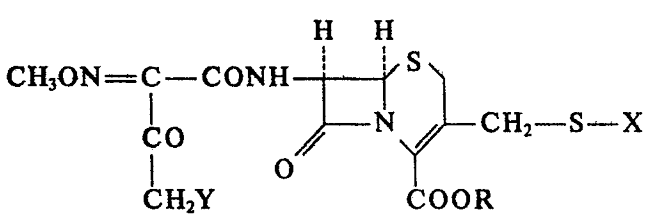

(II)

in which R has the significance given above and Y represents a halogen atom, or a salt of this compound with thiourea and, if desired, converting a compound of formula I obtained

into a salt or hydrate or into a hydrate of this salt, characterized in that X signifies one of the groups

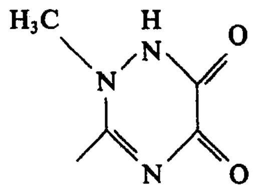
(a)

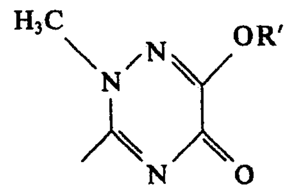
(b)

in which R′ represents hydrogen or a readily hydrolyzable ether group.

2. A process according to claim 1, wherein R and R′ represent hydrogen.

3. A process according to claim 1 or 2, wherein Y represents bromine.

4. A process according to any one of claims 1—3, wherein the starting compound of formula II is present in the syn-form (Z-form).

5. A process according to any one of claims 1—4, wherein the halide of formula II has been prepared by reacting a primary amine of general formula III

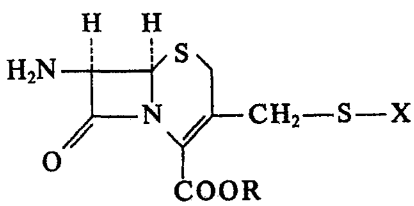
(III)

in which X and R have the significance given in claim 1,
with a halogenated carboxylic acid of general formula IV

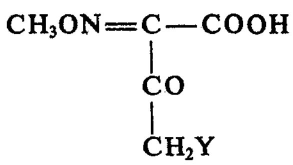
(IV)

in which Y represents a halogen atom,
or with a reactive derivative of this compound.

6. A process according to claim 5, wherein R and R′ represent hydrogen.

7. A process according to claim 5 or 6, wherein Y represents bromine.

8. A process according to any one of claims 5—7, wherein the halogenated carboxylic acid of formula IV or the reactive derivative thereof is present in the syn-form (Z-form).

9. A process according to any one of claims 5—8, wherein an acid chloride of the halogenated carboxylic acid of formula IV is used.

10. A process according to any one of claims 1—4, wherein the halide of formula II has been prepared by halogenating a compound of general formula V

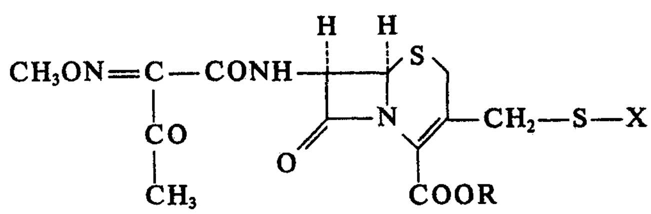
(V)

in which X and R have the significance given in claim 1.

11. A process according to claim 10, wherein R and R′ represent hydrogen.

12. A process according to claim 10 or 11, wherein the compound of formula V is present in the syn-form (Z-form).

13. Compounds of the general formula

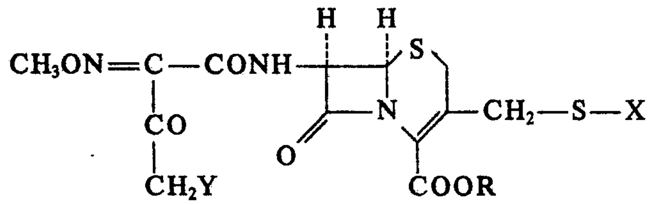

(II)

in which Y signifies a halogen atom and X signifies one of the groups

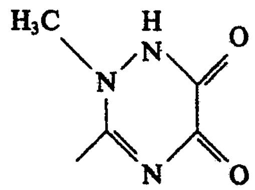

(a)

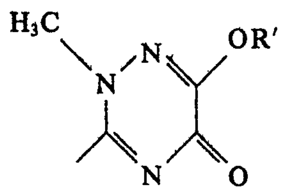

(b)

and R represents hydrogen or a readily hydrolyzable ester group and R′ represents hydrogen or a readily hydrolyzable ether group,
as well as their salts.

14. Compounds in accordance with claim 13, characterized in that R and R′ represent hydrogen.

15. Compounds in accordance with claim 13 or 14, characterized in that Y represents bromine.

16. (6R,7R)-{4-Bromo-2-[(Z)-methoxyimino]acetoacetamido}-3-{[(2,5-dihydro-6-hydroxy-2-methyl-5-oxo-as-triazin-3-yl)thio]methyl}-8-oxo-5-thia-1-azabicyclo[4.2.0]oct-2-ene-2-carboxylic acid and salts thereof.

**Claims for the Contracting State: AT**

1. A process for the manufacture of acyl derivatives of general formula I

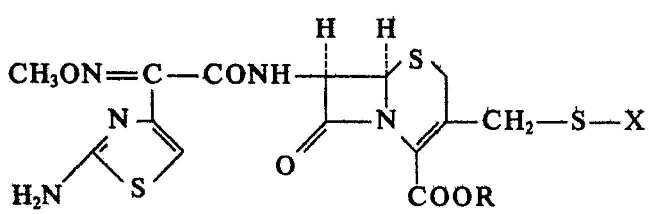

(I)

in which R represents hydrogen or a readily hydrolyzable ester group,
as well as of salts of these compounds and hydrates of these compounds or of their salts by reacting a halide of general formula II

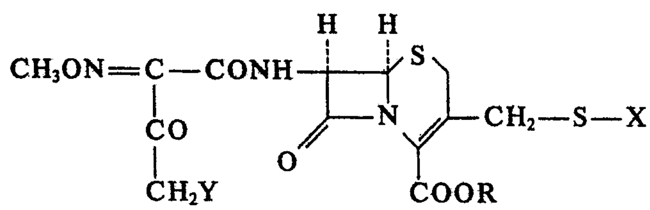

(II)

in which R has the significance given above and Y represents a halogen atom,
or a salt of this compound with thiourea and, if desired, converting a compound of formula I obtained

into a salt or hydrate or into a hydrate of this salt, characterized in that X signifies one of the groups

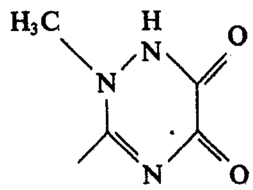

(a)

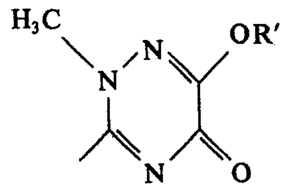

(b)

in which R′ represents hydrogen or a readily hydrolyzable ether group.

2. A process according to claim 1, wherein R and R′ represent hydrogen.

3. A process according to claim 1 or 2, wherein Y represents bromine.

4. A process according to any one of claims 1—3, wherein the starting compound of formula II is present in the syn-form (Z-form).

5. A process according to any one of claims 1—4, wherein the halide of formula II has been prepared by reacting a primary amine of general formula III

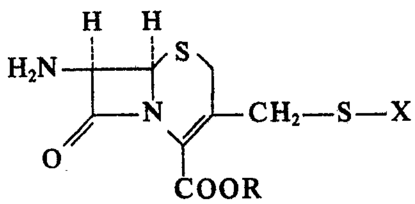

(III)

in which X and R have the significance given in claim 1,
with a halogenated carboxylic acid of general formula IV

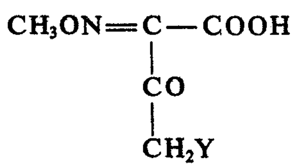

(IV)

in which Y represents a halogen atom,
or with a reactive derivative of this compound.

6. A process according to claim 5, wherein R and R′ represent hydrogen.

7. A process according to claim 5 or 6, wherein Y represents bromine.

8. A process according to any one of claims 5—7, wherein the halogenated carboxylic acid of formula IV or the reactive derivative thereof is present in the syn-form (Z-form).

9. A process according to any one of claims 5—8, wherein an acid chloride of the halogenated carboxylic acid of formula IV is used.

10. A process according to any one of claims 1—4, wherein the halide of formula II has been prepared by halogenating a compound of general formula V

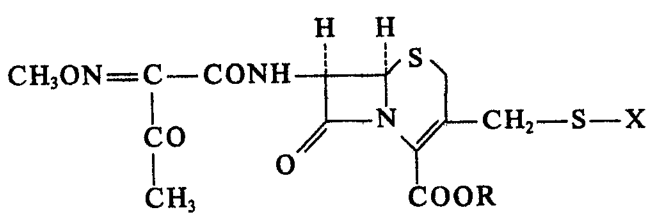

(V)

in which X and R have the significance given in claim 1.

11. A process according to claim 10, wherein R and R′ represent hydrogen.

12. A process according to claim 10 or 11, wherein the compound of formula V is present in the syn-form (Z-form).

**Revendications pour les Etats contractants: BE, CH, DE, FR, GB, IT, LI, LU, NL, SE**

1. Procédé de préparation de dérivés acylés de formule générale I

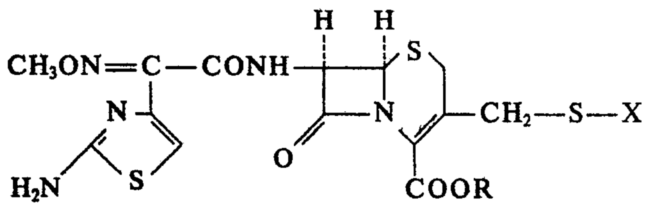

(I)

où R représente un hydrogène ou un groupe ester facilement hydrolysable, ainsi que des sels de ces compoés et des hydrates de ces composés ou de leurs sels par réaction d'un halogénure de formule générale II

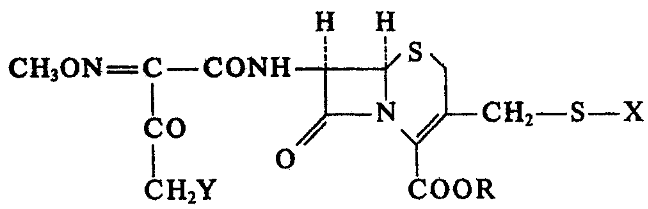

(II)

où R a la signification donnée ci-dessus et Y représente un atome d'halogène, ou d'un sel de ce composé avec de la thiourée et, si on le désire, transformation d'un composé de formule I obtenu en un sel ou un hydrate, ou selon les cas, un hydrate de ce sel, caractérisé en ce que X représente l'un des groupes

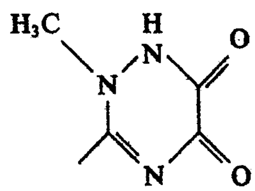

(a)

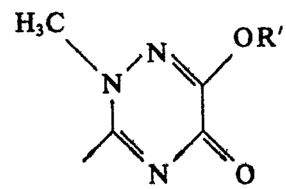

(b)

où R' représente un hydrogène ou un groupe éther facilement hydrolysable.

2. Procédé selon la rev. 1, où R et R' représentent un hydrogène.

3. Procédé selon l'une des rev. 1 ou 2, où Y représente un brome.

4. Procédé selon l'une des rev. 1—3, où le composé de départ de formule II se présente sous la forme syn (forme Z).

5. Procédé selon l'une des rev. 1—4, où l'on prépare l'halogénure de formule II par réaction d'une amine primaire d'une amine primaire de formule générale III

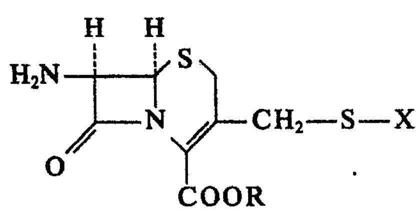

(III)

où X et R ont la signification donnée dans la rev. 1, avec un acide carboxylique halogéné de formule générale IV

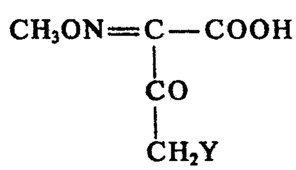

(IV)

où Y représente un atome d'halogène, ou avec un dérivé de ce composé.

6. Procédé selon la revendication 5, où R et R′ représentent un hydrogène.

7. Procédé selon l'une des rev. 5 ou 6, où Y représente un brome.

8. Procédé selon l'une des rev. 5—7, où l'acide carboxylique halogéné de formule IV ou selon les cas son dérivé réactif se présente sous forme syn (forme Z).

9. Procédé selon l'une des rev. 5—8, où l'on utilise un chlorure d'acide de l'acide carboxylique halogéné de formule IV.

10. Procédé selon l'une des rev. 1—4, où l'on prépare l'halogénure de formule II par halogénation d'un composé de formule générale V

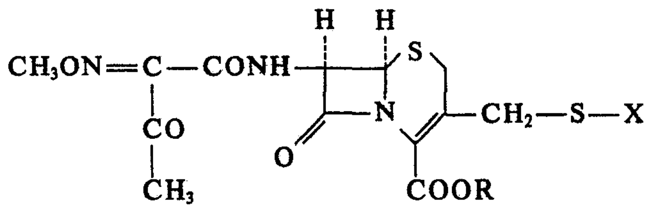

(V)

où X et R ont la signification donnée dans la rev. 1.

11. Procédé selon la rev. 10, où R et R′ représentent un hydrogène.

12. Procédé selon l'une des rev. 10 ou 11, où le composé de formule V se présente sous forme syn (forme Z).

13. Composés de formule générale

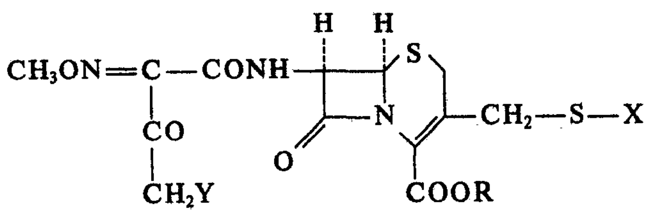

(II)

où Y représente un atome d'halogène et X un des groupes

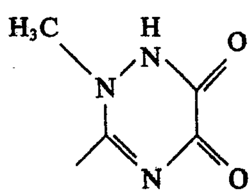

(a)

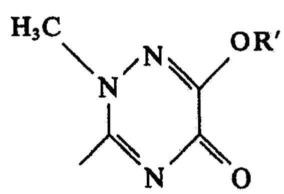

(b)

et R représente un hydrogène ou un groupe ester facilement hydrolysable et R′ représente un hydrogène ou un groupe éther facilement hydrolysable, ainsi que leurs sels.

14. Composés selon la rev. 13, caractérisés en ce que R et R′ représentent un hydrogène.

15. Composés selon l'une des rev. 13 ou 14, caractérisés en ce que Y représente un brome.

16. Acide (6R,7R){4-Bromo-2-[(Z)-méthoxyimino]acétoacétamido}-3-{[(2,5-dihydro-6-hydroxy-2-méthyl-5-oxo-as-tria-zin-3-yl)thio]méthyl}-8-oxo-5-thia-1-azabicyclo[4.2.0]oct-2-ène-2-carboxylique, et ses sels.

**Revendications pour l'Etat contractant: AT**

1. Procédé de préparation de dérivés acylés de formule générale I

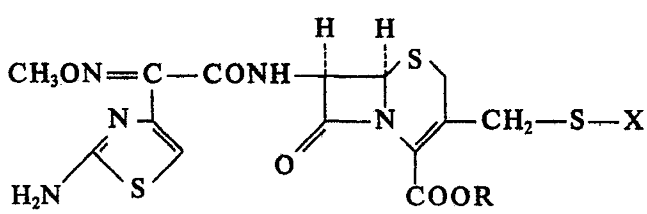

(I)

où R représente un hydrogène ou un groupe ester facilement hydrolysable, ainsi que de sels de ces composés et hydrates de ces composés, ou selon les cas, de leurs sels par

réaction d'un halogénure de formule générale II

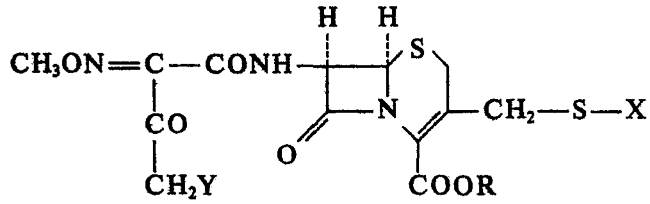

(II)

où R a la signification donnée ci-dessus et Y représente un atome d'halogène, ou d'un sel de ce composé avec de la thiourée et, si on le désire, transformation d'un composé de formule I obtenu en un sel ou hydrate, ou selon les cas, en un hydrate de ce sel, caractérisé en ce que X représente l'un des groupes

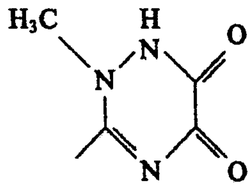

(a)

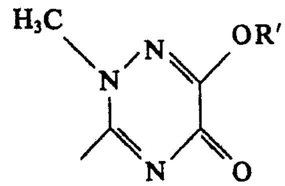

(b)

où R' représente un hydrogène ou un groupe éther facilement hydrolysable.

2. Procédé selon la rev. 1, où R et R' représentent un hydrogène.

3. Procédé selon l'une des rev. 1 ou 2, où Y représente un brome.

4. Procédé selon l'une des rev. 1—3, où le produit de départ de formule II se présente sous forme syn (forme Z).

5. Procédé selon l'une des rev. 1—4, où l'on prépare l'halogénure de formule II par réaction d'une amine primaire de formule III

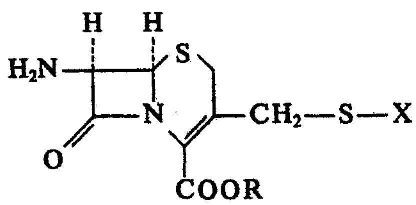

(III)

où X et R ont la signification donnée dans la rev. 1, avec un acide carboxylique halogéné de formule générale IV

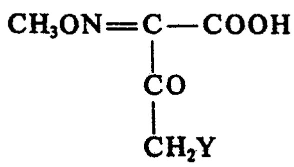

(IV)

où Y représente un atome d'halogène, ou avec un dérivé réactif de ce composé.

6. Procédé selon la rev. 5, où R et R' représentent un hydrogène.

7. Procédé selon l'une des rev. 5 ou 6, où Y représente un brome.

8. Procédé selon l'une des rev. 5—7, où l'acide carboxylique halogéné de formule IV, ou selon les cas, son dérivé réactif, se présente sous forme syn (forme Z).

9. Procédé selon l'une des rev. 5—8, où l'on utilise un chlorure d'acide de l'acide carboxylique halogéné de formule IV.

10. Procédé selon l'une des rev. 1—4, où l'on prépare l'halogénure de formule II par halogénation d'un composé de formule générale V

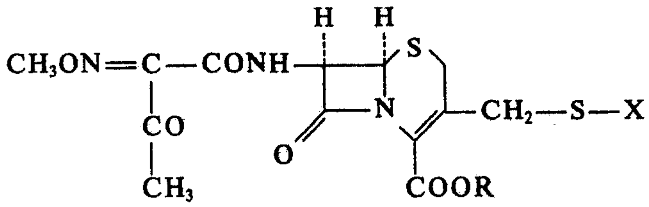

(V)

où X et R ont la signification donnée dans la rev. 1.

11. Procédé selon la rev. 10, où R et R' représentent un hydrogène.

12. Procédé selon l'une des rev. 10 ou 11, où le procédé de formule V se présente sous forme syn (forme Z).